# EUROPÄISCHE PATENTSCHRIFT

(11) **EP 0 554 767 B1**
(45) Veröffentlichungstag und Bekanntmachung des Hinweises auf die Patenterteilung: **19.06.1996**
(21) Anmeldenummer: 93101160.5
(22) Anmeldetag: 27.01.1993
(51) Int. Cl.: C07C 251/48, C07C 59/84, C07C 59/68

(54) **Verfahren zur Herstellung von E-Oximethern von Phenylglyoxylsäureestern**
Process for the preparation of E-oximethers from phenylglyoxylic acid esters
Procédé de préparation d'E-oximéthers d'esters d'acides phénylglyoxyliques

(30) Priorität: 05.02.1992 DE 4203170
(43) Veröffentlichungstag der Anmeldung: 11.08.1993
(73) Patentinhaber: BASF Aktiengesellschaft, D-67063 Ludwigshafen (DE)
(72) Erfinder: Isak, Heinz, Dr., W-6704 Mutterstadt (DE); Keil, Michael, Dr., W-6713 Freinsheim (DE); Wolf, Bernd, Dr., W-6701 Fussgoenheim (DE); Wingert, Horst, Dr., W-6800 Mannheim 1 (DE)

(56) Entgegenhaltungen:
- EP-A- 0 253 213
- EP-A- 0 254 426
- EP-A- 0 386 561
- EP-A- 0 400 417
- EP-A- 0 493 711

## Beschreibung

Die vorliegende Erfindung betrifft ein Verfahren zur Herstellung von E-Oximethern von Phenylglyoxylsäureestern der allgemeinen Formel I, 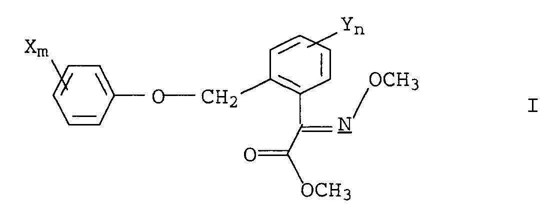
wobei die Substituenten die folgenden Bedeutungen haben:
- X, Y: sind gleich oder verschieden und bedeuten Halogen, C₁-C₄-Alkyl, C₁-C₄-Alkoxy, Trifluormethyl, 1-C₁-C₅-Alkyl-(C₂-C₅-Alkenyl)hydroxyimino-C₁-C₅-alkyl (C₂-C₅-Alkenyl) und 1-C₁-C₅-Alkyl-(C₂-C₅-alkenyl)hydroxyimino
- m: eine ganze Zahl von O bis 4;
- n: eine ganze Zahl von 0 bis 3.

Halogen ist z.B. Fluor, Chlor, Brom, Jod; C₁-C₄-Alkyl ist z.B. Methyl, Ethyl, Propyl (n-Propyl, iso-Propyl), Butyl; C₁-C₄-Alkoxy ist z.B. Methoxy, Ethoxy, Propoxy, Butoxy. 1-C₁-C₅-Alkyl-(C₂-C₅-Alkenyl)-hydroxyimino-C₁-C₅-alkyl-(C₂-C₅-alkenyl) ist z.B. Methylhydroxyimino-methyl (CH₃ON=CCH₃-). 1-C₁-C₅-Alkyl-(C₂-C₅-Alkenyl)hydroxyimino ist z.B. Methylhydroxyimino (CH₃ON=CH-).

Es ist bekannt, Oximether vom Typ der Verbindungen I durch Umsetzung von Glyoxylsäureestern mit O-Methylhydroxylaminhydrochlorid herzustellen (vgl. z. B. EP-A 253 213 und EP-A 254 426), wobei als Nebenprodukt äquimolare Mengen an Chlorwasserstoff gebildet werden. Nachteilig bei diesem Verfahren ist jedoch, daß E/Z-Isomerengemische der Oximether entstehen, die nur mit relativ großem technischem Aufwand getrennt werden können. Bei diesem Verfahren erhält man das bevorzugte Isomere mit der E-Konfiguration an der Oximbindung nur in sehr geringen Mengen.

Ferner wird ein Herstellungsverfahren beschrieben (EP-A-493,711), in dem eine an die Herstellung anschließende Isomerisierung zu einem hohen Anteil an dem gewünschten Isomeren mit E-Konfiguration führt. Nachteilig an diesem Verfahren ist jedoch zum einen der große Überschuß an Chlorwasserstoff, der zur Isomerisierung benötigt wird, sowie eine Belastung der Wirtschaftlichkeit des Verfahrens durch die Verwendung des schwierig zugänglichen Methoxyaminhydrochlorids.

Es ist aus Houben-Weyl, Methoden der Organischen Chemie, Band 10/4, Seiten 217 bis 223 bekannt, daß bei der Umsetzung von Ketoximen mit einem Alkylierungsmittel die Alkylgruppe sowohl an den Stickstoff des Oxims als auch an den Sauerstoff des Oxims gebunden werden kann. Im ersten Fall erhält man ein Nitron, im zweiten Fall einen Oximäther. Meistens findet man beide Reaktionsprodukte nebeneinander. Es werden Methoden beschrieben, mit deren Hilfe man diese Endstoffe ausschließlich erhalten kann, doch sind diese Methoden entweder großtechnisch nicht durchführbar oder sie liefern den gewünschten Stoff nur in ungenügender Ausbeute. So findet sich in Houben-Weyl (loc. cit., Seite 223) der Hinweis, daß das getrocknete Silbersalz des jeweiligen Oxims in absolutem Äther oder Alkohol mit Alkyljodid in Gegenwart von Silberoxid umgesetzt werden kann. Diese Arbeitsweise ist umständlich und großtechnisch unwirtschaftlich. Man kann auch Cyclohexanonoxim in wäßriger Natronlauge mit Dimethylsulfat methylieren (Coll. Czech. Chem. Comm. 14, 561 - 563 (1949)). Die Ausbeute beträgt aber nur 37 % der Theorie an noch unreinem Endstoff. Auch im Falle der Umsetzung des Oxims mit Allylbromid erhält man lediglich eine Ausbeute von 35,9 % der Theorie.

Darüber hinaus ist aus EP 23560 ein Verfahren zur Herstellung von o-substituierten Ketoximen bekannt. In dieser Veröffentlichung werden jedoch die Oxime von Phenylglyoxylsäureestern nicht beschrieben.

Aufgabe der vorliegenden Erfindung war es deshalb, die Verbindungen I besser zugänglich zu machen.

Demgemäß wurde ein Verfahren zur Herstellung von E-Oximethern der Formel I gefunden, welches dadurch gekennzeichnet ist, daß man einen o-Phenoxymethylphenylglyoxylsäureester der allgemeinen Formel IVa oder ein Ketal der allgemeinen Formel IVb oder ein Amid der allgemeinen Formel IVc oder ein Gemisch der Verbindungen IVa, IVb und IVc mit Hydroxylamin oder einem seiner Säureadditionssalze zunächst in ein Gemisch der E- und Z-Oxime der allgemeinen Formeln IIa und IIb überführt, das so erhaltene Gemisch der E- und Z- Oxime anschließend durch Behandlung mit einer Säure in einem organischen Lösungsmittel in das E-Oxim der Formel IIa überführt, IIa anschließend in Gegenwart eines organischen Verdünnungsmittel mit einer Base in das entsprechende Salz überführt und dieses mit einem Methylierungsmittel der allgemeinen Formel III in der X für Chlor, Brom, Iod, CH₃OSO₃, CF₃OSO₃, (4-CH₃-C₆H₄O)-SO₃ oder (4-Br-C₆H₄O)-SO₃ steht, in I überführt. 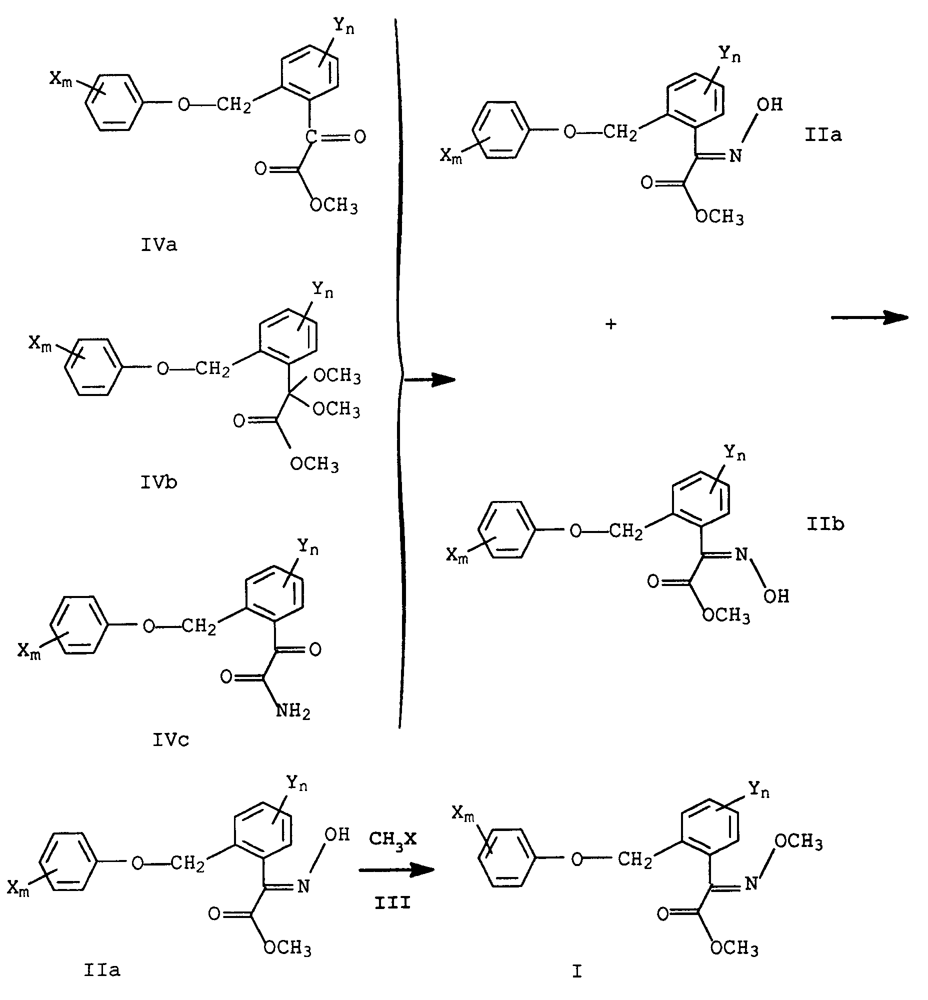
Die E-Oxime von Phenylglyoxylsäureestern IIa werden bei dem erfindungsgemäßen Verfahren in Gegenwart eines organischen Verdünnungsmittels mit einer Base in das entsprechende Salz überführt und dieses mit einer Verbindung der Formel III umgesetzt.

Die Umsetzung wird im allgemeinen bei einer Temperatur von -20°C bis +100°C, vorzugsweise von 0°C bis 80°C, insbesondere bei 20°C bis 80°C, drucklos oder unter Druck, kontinuierlich oder diskontinuierlich durchgeführt.

Man setzt die Ausgangsstoffe in stöchiometrischer Menge oder im Überschuß zweckmäßig in einer Menge von 1 bis 2, insbesondere von 1,05 bis 1,5 Mol Ausgangsstoff III und 1 bis 1,5 Mol Base, je Mol Ausgangsstoff II, um. Als organische Verdünnungsmittel sind aprotisch-dipolare Lösungsmittel brauchbar. Als aprotisch-dipolare Lösungsmittel werden hier solche Lösungsmittel definiert, bei denen ein Lösungsmittelmolekül ein ausgeprägtes Dipolmoment besitzt, jedoch keine Wasserstoffatome trägt, welche zur Ausbildung von Wasserstoffbrücken befähigt sind. Die Dielektrizitätskonstante solcher Lösungsmittel ist größer als 15. Bezüglich der Definition für aprotisch-dipolare Lösungsmittel wird auf A. J. Parker, Chem. Rev. 69 (1969), Seiten 1 - 32, insbesondere Seite 2, verwiesen. So sind beispielsweise geeignete aprotisch-dipolare Lösungsmittel Sulfoxide wie Dimethylsulfoxid, Diäthylsulfoxid, Dimethylsulfon, Diäthylsulfon, Methyläthylsulfon, Tetramethylensulfon; Nitrile wie Acetonitril, Benzonitril, Butyronitril, Isobutyronitril, m-Chlorbenzonitril; N,N-disubstituierte Carbonamide wie Dimethylformamid, Tetramethylharnstoff, N,N-Dimethylbenzamid, N,N-Dimethylacetamid, N,N-Dimethylphenylacetamid, N,N-Dimethylcyclohexancarbonsäureamid, N,N-Dimethylpropionsäureamid und homologes Carbonsäurepiperidid, Carbonsäuremorpholid, Carbonsäurepyrrolidid; entsprechende N,N-Diäthyl-, N,N-Dipropyl-, N,N-Diisopropyl-, N,N-Diisobutyl-, N,N-Dibenzyl-, N,N-Diphenyl-, N-Methyl-N-phenyl-, N-Cyclohexyl-N-methyl-, N-Äthyl-N-tert.-butyl-verbindungen, N-Methyl-formanilid, N-Äthylpyrrolidon, N-Butylpyrrolidon, N-Äthyl-piperidon-(6), N-Methylpyrrolidon; Hexamethylphosphorsäuretriamid; und entsprechende Gemische.Bevorzugt sind Dimethylacetamid, N-Methylpyrrolidon, Dimethylformamid, Dimethylsulfoxid und Tetramethylensulfon. Besonders bevorzugt N-Methylpyrrolidon und Dimethylformamid.

Eine Verfahrensvariante besteht darin, daß das Oxim-Salz ohne Abtrennung des Verdünnungsmittels weiter umgesetzt wird.

Das E-Oxim der Formel IIa erhält man, wenn man eine Mischung aus dem E-Oxim der Formel IIa und dem Z-Oxim der Formel IIb mit einer Säure in einem organischen Verdünnungsmittel behandelt. Ein organisches Verdünnungsmittel ist z.B. ein aromatischer Kohlenwasserstoff oder ein chlorierter Kohlenwasserstoff oder ein Alkohol.

Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol und Ethanol. Besonders bevorzugt ist Methanol.

Als Säuren eignen sich insbesondere Mineralsäuren, beispielsweise Perchlorsäure, Schwefelsäure, Phosphorsäure und Halogenwasserstoffsäuren wie Chlorwasserstoff, aliphatische Sulfonsäuren wie Trifluormethansulfonsäure, aromatische Sulfonsäuren wie p-Toluolsulfonsäure sowie halogenierte Alkancarbonsäuren wie Trifluoressigsäure. Besonders bevorzugt ist Chlorwasserstoff-Gas.

Üblicherweise verwendet man die 0,01fache bis 10fache, insbesondere die 0,01 bis 5fache, molare Menge an Säure, bezogen auf die Menge der Mischung aus IIa und IIb.

Die Reaktionstemperatur für die Isomerisierung liegt im allgemeinen zwischen (-20) und 100°C, insbesondere zwischen 20 und 80°C.

Die Umlagerung der Oxime benötigt eine gewisse Zeit und zwar abhängig von der Temperatur und insbesondere der Säuremenge etwa 1 bis 90 Stunden, vorzugsweise 2 bis 10 Stunden.

In der Regel können alle genannten Verfahrensschritte bei Atmosphärendruck oder unter dem Eigendruck des jeweiligen Systems, bis ca. 5 bar, ausgeführt werden. Höherer oder niedrigerer Druck ist auch möglich, bietet im allgemeinen jedoch keine Vorteile.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Die Mischung aus dem E-Oxim der Formel IIa und dem Z-Oxim der Formel IIb erhält man beispielsweise, wenn man einen entsprechenden Phenylglyoxylsäureester der Formel IVa mit Hydroxylamin oder einem seiner Säureadditionssalze umsetzt.

Das Hydroxylamin wird entweder in Form eines Säureadditionssalzes oder als freie Base eingesetzt, wobei die unprotonierte Verbindung durch Zugabe einer starken Base aus dem Salz freigesetzt werden kann. Als Salze des Hydroxylamins kommen die Salze mit ein- bis dreiwertigen Säuren, wie insbesondere Salzsäure und Schwefelsäure, in Betracht.

Beispielsweise nimmt man die Reaktion in Gegenwart eines Lösungs- oder Verdünnungsmittels vor.

Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol und Ethanol. Besonders bevorzugt ist Methanol.

Die Mengenverhältnisse der Ausgangsprodukte sind nicht kritisch; zweckmäßigerweise setzt man stöchiometrische Mengen an Ausgangsverbindungen ein, sofern sich nicht ein Überschuß der einen oder anderen Komponente, z.B. 10 mol-% empfiehlt.

Normalerweise liegt die Reaktionstemperatur zwischen 0 und 100°C, vorzugsweise zwischen 20 und 80°C.

Die E-Oxime der Formel IIa sind auch dadurch erhältlich, daß man einen Phenylglyoxylsäureester der allgemeinen Formel IVa 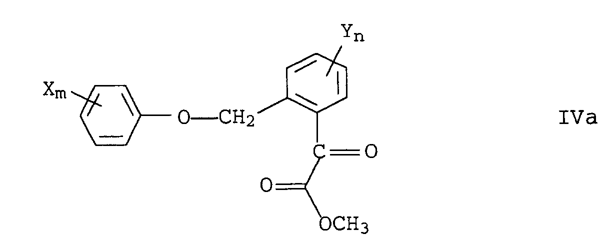
oder ein Ketal eines Phenylglyoxylsäureesters der allgemeinen Formel IVb 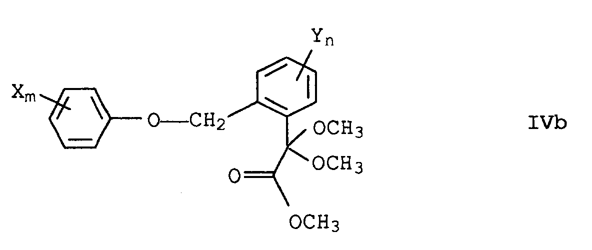
oder ein Amid der allgemeinen Formel IVc 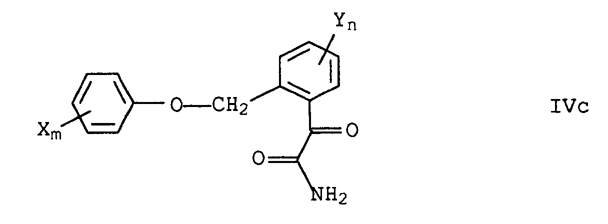
oder ein Gemisch aus den Verbindungen IVa, b, c mit Hydroxylamin oder einem seiner Säureadditionssalze umsetzt und gleichzeitig oder anschließend mit einer Säure in einem organischen Verdünnungsmittel behandelt.

Die als Ausgangsstoffe dienenden Phenylglyoxylsäureester, Ketale und Amide der Formel IVa-c sind beispielsweise nach dem in EP-A-493 711 beschriebenen Verfahren erhältlich.

Sowohl die Phenylglyoxylsäureester IVa als auch die Derivate IVb und IVc oder Gemische dieser Verbindungen eignen sich als Ausgangsstoffe für das erfindungsgemäße Verfahren. Insbesondere kann das durch eine Pinner-Reaktion erhaltene Rohprodukt-Gemisch IVa-c ohne weitere Reinigung nach diesem Verfahren in das E-Oxim der Formel IIa überführt werden.

Das Hydroxylamin wird entweder in Form eines Säureadditionssalzes oder als freie Base eingesetzt, wobei die unprotonierte Verbindung durch Zugabe einer starken Base aus dem Salz freigesetzt werden kann. Als Salze des Hydroxylamins kommen die Salze mit ein- bis dreiwertigen Säuren, wie insbesondere Salzsäure und Schwefelsäure, in Betracht.

Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol und Ethanol. Besonders bevorzugt ist Methanol.

Die Mengenverhältnisse der Ausgangsprodukte sind nicht kritisch; zweckmäßigerweise setzt man stöchiometrische Mengen an Ausgangsverbindungen ein, sofern sich nicht ein Überschuß der einen oder anderen Komponente, z.B. 10 mol-% empfiehlt.

Normalerweise liegt die Reaktionstemperatur zwischen 0 und 100°C, vorzugsweise zwischen 20 und 80°C.

Eine Verfahrensvariante besteht darin, die aus der Pinner-Reaktion erhaltene Mischung aus den Verbindungen IVa und IVb ohne Abtrennung aus der Reaktionsmischung mit Hydroxylamin oder einem seiner Säureadditionssalze umzusetzen.

Die Oxime von Phenylglyoxylsäureestern werden in der Regel als Isomerengemische erhalten, wobei der Oximrest teilweise in der E- und teilweise in der Z-Konfiguration vorliegt. Die Umlagerung der Oxime in die E-Konfiguration erfolgt durch Behandlung mit einer Säure.

Zu diesem Zweck kann die Rohlösung der Oxime zuvor eingeengt oder weiter verdünnt werden. Gewünschtenfalls kann die Umlagerung auch in einem 2-Phasensystem aus Wasser/Säure und einem organischen Lösungsmittel wie Dichlormethan erfolgen. Zweckmäßig behandelt man die erhaltene Rohlösung des Oxims jedoch ohne weitere Aufkonzentration oder Verdünnung direkt mit der Säure. Als Säuren eignen sich insbesondere Mineralsäuren, beispielsweise Perchlorsäure, Schwefelsäure, Phosphorsäure und Halogenwasserstoffsäuren wie Chlorwasserstoff, aliphatische Sulfonsäuren wie Trifluormethansulfonsäure, aromatische Sulfonsäuren wie p-Toluolsulfonsäure sowie halogenierte Alkancarbonsäuren wie Trifluoressigsäure. Besonders bevorzugt ist Chlorwasserstoff-Gas.

Überlicherweise verwendet man die 0,01fache bis 10fache, insbesondere die 0,01 bis 5fache, molare Menge an Säure, bezogen auf die Menge der Mischung aus IIa und IIb.

Die Reaktionstemperatur für die Isomerisierung liegt im allgemeinen zwischen (-20) und 100°C, insbesondere zwischen 20 und 80°C.

Die Umlagerung der Oxime benötigt eine gewisse Zeit und zwar abhängig von der Temperatur und insbesondere der Säuremenge etwa 1 bis 90 Stunden, vorzugsweise 2 bis 10 Stunden.

In der Regel können alle genannten Verfahrensschritte bei Atmosphärendruck oder unter dem Eigendruck des jeweiligen Systems, bis ca. 5 bar, ausgeführt werden. Höherer oder niedrigerer Druck ist auch möglich, bietet im allgemeinen jedoch keine Vorteile.

Das erfindungsgemäße Verfahren kann sowohl diskontinuierlich als auch kontinuierlich durchgeführt werden. Bei der kontinuierlichen Arbeitsweise leitet man die Reaktionspartner beispielsweise durch einen Rohrreaktor oder über Rührkesselkaskaden.

Das erfindungsgemäße Verfahren läßt sich mit Erfolg zur Synthese der E-Oxime von Phenylglyoxylsäureestern anwenden, vor allem für solche Verbindungen, in denen die Substituenten X und Y unabhängig voneinander die folgende Bedeutung haben:
- Halogen wie Fluor, Chlor und Brom;
- verzweigtes oder unverzweigtes C₁-C₄-Alkyl wie Methyl, Ethyl, Isopropyl und n-Butyl, insbesondere Methyl und Ethyl;
- C₁-C₄-Alkoxy wie Methoxy, Ethoxy, 1-Methylethoxy und n-Propoxy;
- Trifluormethyl.

Die E-Oxime der Formel IIa werden nach dem erfindungsgemäßen Verfahren überraschenderweise in hoher Ausbeute und ausgezeichneter Reinheit zugänglich. Im Hinblick auf den bekannten Stand der Technik war dagegen zu erwarten, daß bei dem Verfahren die gleichen Schwierigkeiten auftreten, wie bei den bekannten Herstellverfahren. Insbesondere war keinesfalls vorauszusehen, daß unter sauren Bedingungen eine Isomerisierung mit überwiegender Bildung des E-Isomeren stattfindet, da bei den bekannten Synthesemethoden bei der Verwendung von Hydroxylaminhydrochlorid während der Umsetzung äquimolare Mengen an Chlorwasserstoff entstehen, ohne daß ein Isomeres bevorzugt entsteht. Weiterhin hätte man bei Behandlung der Rohprodukte mit Säure Zersetzungsreaktionen des Oxims erwarten müssen.

Das erfindungsgemäße Verfahren besitzt eine Reihe von Vorteilen:
- es ist in technischem Maßstab in sehr einfacher Weise durchführbar;
- die Salze des Hydroxylamins können als wäßrige Lösungen eingesetzt werden;
- der Phenylglyoxylsäureester IVa kann als Rohprodukt aus der Vorstufe eingesetzt werden, da das als Verunreinigung enthaltene Dimethylketal des Phenylglyoxylsäureesters überraschenderweise auch in das gewünschte E-Oxim überführt wird.

Die E-Oximether der Formel I werden in EP-A 253 213 und der EP-A 254 426 als Pflanzenschutzmittel beschrieben.

Bei der Umsetzung der Verbindungen IIa mit Alkylierungsmitteln der Formel III können neben den Verbindungen I Nitrone der allgemeinen Formel V 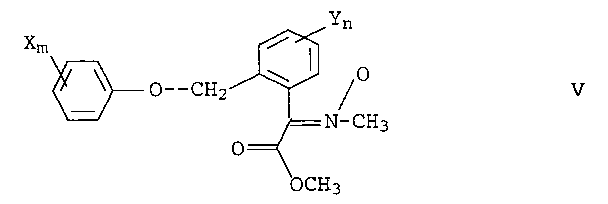
gebildet werden.

Die Nitrone V lassen sich durch Umsetzung mit O-Methylhydroxylamin oder einem seiner Säureadditionssalze bei einer Temperatur von -20°C bis +100°C, vorzugsweise von 0°C bis 80°C, insbesondere bei 20°C bis 80°C, in die Verbindungen I überführen. Die Umsetzung erfolgt drucklos oder unter Druck, kontinuierlich oder diskontinuierlich.

Das Methylhydroxylamin wird entweder in Form eines Säureadditionssalzes oder als freie Base eingesetzt, wobei die unprotonierte Verbindung durch Zugabe einer starken Base aus dem Salz freigesetzt werden kann. Als Salze des Methylhydroxylamins kommen die Salze mit ein- bis dreiwertigen Säuren, wie insbesondere Salzsäure und Schwefelsäure, in Betracht.

Beispielsweise nimmt man die Reaktion in Gegenwart eines Lösungs- oder Verdünnungsmittels vor.

Geeignete Lösungsmittel sind vorzugsweise aromatische Kohlenwasserstoffe wie Benzol, Toluol und o-, m-, p-Xylol, chlorierte Kohlenwasserstoffe wie Methylenchlorid, Alkohole wie Methanol und Ethanol. Besonders bevorzugt ist Methanol.

Die Mengenverhältnisse der Ausgangsprodukte sind nicht kritisch; zweckmäßigerweise setzt man stöchiometrische Mengen an Ausgangsverbindungen ein, sofern sich nicht ein Überschuß der einen oder anderen Komponente, z.B. 10 mol-% empfiehlt.

Die Nitrone der Formel V werden auch bei der Umsetzung der Mischung der Verbindungen IIa und IIb mit Alkylierungsmitteln der Formel III gebildet.

Sie können durch Umsetzung mit Hydroxylamin oder einem seiner Säureadditionssalze in die E-Oximether der Formel IIa überführt werden.

Die Umsetzung wird im allgemeinen bei einer Temperatur von -20° bis 100°C, vorzugsweise von 0 bis 80°C, insbesondere bei 20 bis 80°C, drucklos oder unter Druck, kontinuierlich oder diskontinuierlich durchgeführt.

Als organische Lösungsmittel sind aprotisch-dipolare und protische Lösungsmittel brauchbar. Bevorzugt sind Alkohole. Besonders bevorzugt ist Methanol oder ein Methanol/Wasser-Gemisch.

Das Hydroxylamin wird entweder in Form eines Säureadditionssalzes oder als freie Base eingesetzt.

Die Mengenverhältnisse der Ausgangsprodukte sind nicht kritisch, zweckmäßigerweise setzt man stöchiometrische Mengen an Ausgangsverbindungen ein, sofern sich nicht ein Überschuß der einen oder anderen Komponente, z.B. 10 - 20 mol-% empfiehlt.

Die Nitrone der Formel V können daneben in die Glyoxylsäureester der Formel IVa/IVb überführt werden, wenn man sie
c₁) mit einem Alkohol oder Alkoholat, besonders bevorzugt Methanol oder Methanolat und anschließender wäßriger Aufarbeitung umsetzt,
c₂) oder mit einer Säure in Gegenwart eines organischen Verdünnungsmittels vorzugsweise eines Alkohols, besonders bevorzugt Methanol umsetzt.

Die Umsetzungen werden im allgemeinen bei einer Temperatur von -20° bis 100°C, insbesondere bei 20° bis 70°C, drucklos oder unter Druck durchgeführt.

Die Mengenverhältnisse der Ausgangsprodukte sind nicht kritisch, zweckmäßigerweise setzt man stöchiometrische Mengen aus Ausgangsverbindungen ein, sofern sich nicht ein Überschuß der einen oder anderen Komponente, z.B. 30 bis 50 % empfiehlt.

Als Säuren eignen sich insbesondere Mineralsäuren, beispielsweise Perchlorsäure, Schwefelsäure, Phosphorsäure und Halogenwasserstoffsäuren wie Chlorwasserstoff, aliphatische Sulfonsäuren wie Trifluormethansulfonsäure, aromatische Sulfonsäuren wie p-Toluolsulfonsäure sowie halogenierte Alkancarbonsäuren wie Trifluoressigsäure. Besonders bevorzugt ist Chlorwasserstoff-Gas.

### Beispiel 1

### Herstellung von E-2-(2'-Methylphenoxymethyl)-phenylglyoxylsäuremethylester-O-methyloxim

a) Man löst 11,96 g (40 mmol) E-2-(2'-Methylphenoxymethyl)-phenylglyoxylsäuremethylester-Oxim in 40 ml Dimethylformamid, fügt 2,38 g (43 mmol) Natriummethylat zu und rührt 15 min nach. Nun kühlt man auf 0-5°C ab und gast 3,03 g (60 mmol) Methylchlorid ein. Anschließend läßt man bei RT (Raumtemperatur 20°C) 1 h nachrühren.
   HPLC (Hochdruck-Flüssigkeits-Chromatogramm)-Kontrolle: 84 % Produkt, 11 % Nitron (Gew.%)
   Nach Zugabe von 0,5 g Natrium-methylat setzt sich das Nitron um. Das Rohprodukt wird eingeengt, in Methyltertiärbutylether (MTBE) aufgenommen, 3x mit Wasser gewaschen, über Magnesiumsulfat getrocknet und vom Lösungsmittel befreit.
   Ausbeute: 10,1 g (81 %)
   Reinheit: > 95 % (nach HPLC)
b) Beispiel mit DMS (Dimethylsulfat) (Toluol)
   Man löst 15,0 g (47 mmol) E-2(2'-Methylphenoxymethyl)phenylglyoxylsäuremethylester-Oxim in 100 ml Toluol, gibt 9,45 g 30%ige Natriummethylat-Lösung dazu und rührt 15 min nach. Danach gibt man 5,2 ml (55 mmol) Dimethylsulfat zu und rührt ca. 8 h nach. Abschließend engt man ein, nimmt in 20 ml Diethylether auf wäscht 3 x mit Wasser, trocknet über Magnesiumsulfat und engt nochmals ein.
   Ausbeute: 14.1 g (96 %)
   Reinheit: höher als 87 %
c) Zu einer Lösung von 29,9 g (0,1 mol) E-2-(2'-Methylphenoxymethyl)-phenylglyoxylsäuremethylester-oxim in 100 ml 1-Methyl-2-pyrrolidon tropft man bei 25°C 19 ml einer Lösung von Natriummethylat (0,105 mol) in Methanol (30 %ig) und rührt 15 Minuten bei dieser Temperatur. Nach Abdestillieren des Methanols bei vermindertem Druck kühlt man die Reaktionslösung auf -5°C ab und leitet 7,6 g (0,15 mol) Methylchlorid ein. Anschließend wird 5 Stunden bei 0 bis +5°C gerührt. Das Lösungsmittel wird bei vermindertem Druck abdestilliert und der Rückstand mit Diethylether und Wasser aufgenommen. Nach der Phasentrennung extrahiert man die organische Phase noch 3x mit Wasser, trocknet über Natriumsulfat und engt ein. Der verbleibende Rückstand (29 g) enthält 85 % des gewünschten O-Methyloxims neben 11 % 2-(2'-Methylphenoxymethyl)-phenylglyoxylsäuremethylester-methylnitron. Durch Umkristallisation aus Methanol kann das reine O-Methyloxim erhalten werden.

### Beispiel 2

### E/Z-2-(2'-Methylphenoxymethyl)-phenylglyoxylsäuremethylester-Oxim

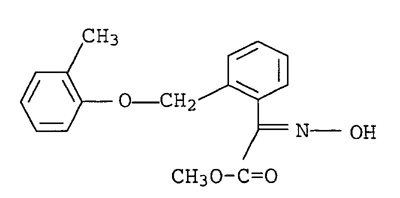

a) 5,68 g (20 mmol) des 2-(2'-Methylphenoxymethyl)-phenylglyoxylsäuremethylesters werden in 25 ml Methanol gelöst und mit 1,53 g (22 mmol) Hydroxylamin-Hydrochlorid versetzt. Das Reaktionsgemisch wird 2 h zum Rückfluß erhitzt. Das Rohprodukt wird vom Lösungsmittel befreit, in ca. 100 ml MTBE gelöst, 3x mit 20 ml Wasser gewaschen, über Natriumsulfat getrocknet und zur Trockne eingeengt.
   Ausbeute: 5,90 g = 100 % d. Theorie
   Reinheit: 92,6 % (cis:trans = 75 : 25)
b) Zu 27 ml Methanol, 135 ml Toluol und 3,6 g Wasser werden bei -5°C 45,2 g (0,18 mol) 2-(2'-Methyl-phenoxymethyl)-benzoylcyanid gegeben. Bei -10°C bis -5°C leitet man 27 g Chlorwasserstoffgas ein, erwärmt auf 25°C und rührt 15 Stunden bei dieser Temperatur nach. Die erhaltene Reaktionsmischung [25 % 2-(2'-Methylphenoxymethyl)-phenylglyoxylsäuremethylester, 63 % 2-(2'-Methylphenoxymethyl)-phenylglyoxylsäureamid] wird mit 121,5 ml Methanol und 12,5 g (0,18 mol) Hydroxylammoniumchlorid versetzt und 7 Stunden bei 64°C gerührt. Nach Abkühlen auf Raumtemperatur saugt man das ausgefalllene Ammoniumchlorid ab und engt die Mutterlauge ein. Der verbleibende Rückstand (54 g) enthält 72 % E-Oxim und 12 % Z-Oxim.

Durch Säulenchromatographie konnten die Isomeren rein erhalten werden.
Isomere:
E-2-(2'-Methylphenoxlmethyl)-phenylglyoxylsäuremethylester-Oxim,
Schmp. 115°C
Z-2(2'-Methylphenoxymethyl)-phenylglyoxylsäuremethylester-Oxim,
Schmp. 105°C

### Beispiel 3

### Herstellung von E-2-(2'-Methylphenoxymethyl)-phenylglyoxylsäuremethylester-oxim

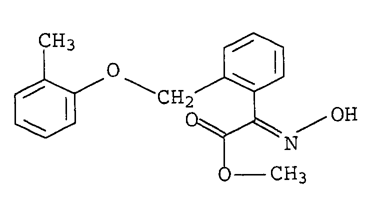

56,8 g (0,2 mol) 2-(2'-Methylphenoxymethyl)-phenylglyoxylsäuremethylester und 15,2 g (0,22 mol) Hydroxylammoniumchlorid werden in 67,5 ml Methanol 6,5 Stunden bei Rückflußtemperatur gerührt. In die erhaltene Reaktionsmischung (E/Z-Oximisomerengemisch) leitet man bei 0°C 14,2 g (0,39 mol) Chlorwasserstoff ein und rührt anschließend 5 Stunden bei 25°C. Das Lösungsmittel wird bei vermindertem Druck abdestilliert, der verbleibende Rückstand mit Diethylether aufgenommen und die erhaltene Lösung mit Wasser gewaschen, über Natriumsulfat getrocknet und zur Trockne eingeengt Man erhält 55,3 g Feststoff (FP. 115°C) = E-Isomeres.

Unter ähnlichen Bedingungen wurden die folgenden Versuche zur Herstellung der gleichen Verbindung durchgeführt.

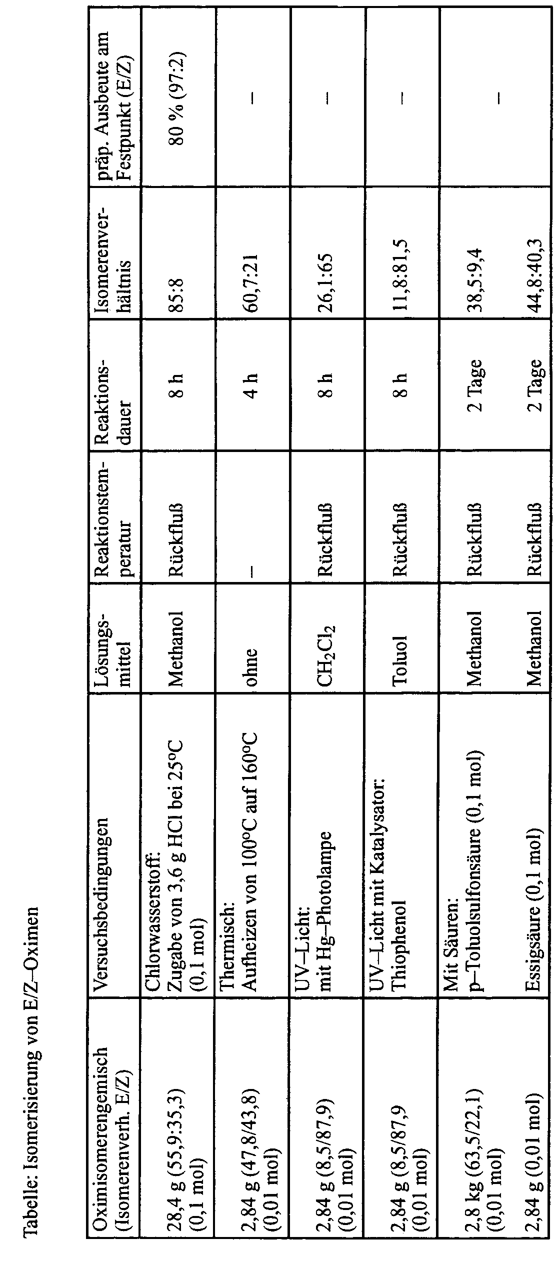

### Beispiel 4

15,7 g eines Produktgemisches, welches 30 % 2-(2'-Methylphenoxymethyl)-phenylglyoxylsäuremethylester-methylnitron und 42 % E-2-(2'-Methylphenoxymethyl)-phenylglyoxylsäuremethylester-O-methyloxim enthält, werden mit 70 ml Methanol versetzt. Nach Zugabe von 0,9 g (0,05 mol) Wasser werden 3,7 g (0,1 mol) Chlorwasserstoff eingeleitet. Man rührt 1 Stunde bei 65°C. Das Lösungsmittel wird abdestilliert, der verbleibende Rückstand in Essigsäureethylester aufgenommen und die organische Lösung 2mal mit Wasser extrahiert. Nach Trocknung über Natriumsulfat wird das Lösungsmittel abdestilliert. Es verbleiben 14,6 g. Die HPLC-Analytik zeigt, daß das Methylnitron vollständig in 2-(2'-Methylphenoxymethyl)-phenylglyoxylsäuremethylester und 2-(2'-Methylphenoxymethyl)-phenylglyoxylsäuremethylester-dimethylketal (mit Verhältnis 3/1) umgewandelt wurde.

### Beispiel 5

a) 1,5 g (4,8 mmol) 2-(2'-Methylphenoxymethyl)-phenylglyoxylsäuremethylester-methylnitron werden in 20 ml Methanol gelöst und mit 3,8 g (5,5 mmol) Hydroxylamin-Hydrochlorid versetzt. Das Reaktionsgemsich wird nun 4 h zum Rückfluß erhitzt und anschließend von Methanol befreit. Danach in 50 ml Diethylether aufgenommen, mit 2 x 20 ml Wasser gewaschen, die Phasen werden getrennt und die organische Phase eingeengt.
   Ausbeute: 1,1 g (3,6 mmol, 75 %; 2-(2'-Methylphenoxymethyl)-phenylglyoxylsäuremethylester-oxim (Isomerengemisch).
b) 1,5 g (4,8 mmol) 2-(2'-Methylphenoxymethyl)-phenylglyoxylsäuremethylester-methylnitron werden in 20 ml Methanol gelöst und mit 4,6 g (5,5 mmol) Methoxyamin-Hydrochlorid versetzt. Das Reaktionsgemisch wird 4 h zum Rückfluß erhitzt und anschließend vom Methanol befreit. Danach nimmt man in 50 ml Dichlormethan auf, wäscht 2 x mit 20 ml Wasser, trennt die Phasen und engt die organische Phase ein.
   Ausbeute: 0.9 g (60 %), 2-(2'-Methylphenoxymethyl)-phenylglyoxylsäuremethylester-O-methyl-oxim (Isomerengemisch).

## Patentansprüche

1. Verfahren zur Herstellung von E-Oximethern der Formel I 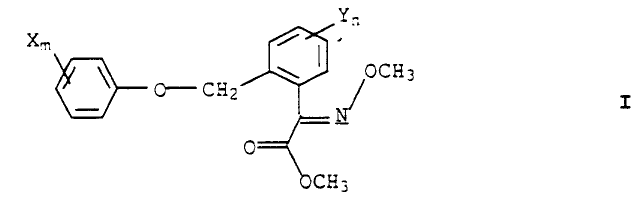 wobei die Substituenten die folgende Bedeutung haben:
X, Y sind gleich oder verschieden und bedeuten Halogen, C₁-C₄-Alkyl, C₁-C₄-Alkoxy, Trifluormethyl, 1-C₁-C₅-Alkyl-(C₂-C₅-Alkenyl)hydroxyimino-C₁-C₅-Alkyl (C₂-C₅-Alkenyl) und 1-C₁-C₅-Alkyl-(C₂-C₅-Alkenyl)hydroxyimino;
m eine ganze Zahl von 0 bis 4;
n eine ganze Zahl von 0 bis 3,
dadurch gekennzeichnet, daß man einen o-Phenoxymethylphenylglyoxylsäureester der allgemeinen Formel IVa 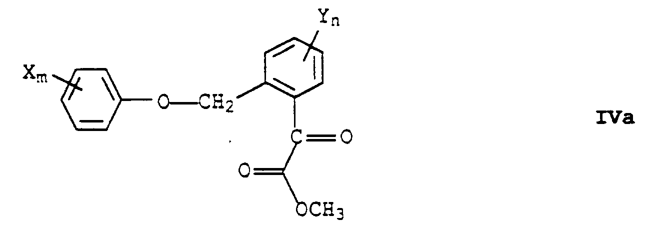 oder ein Ketal der allgemeinen Formel IVb 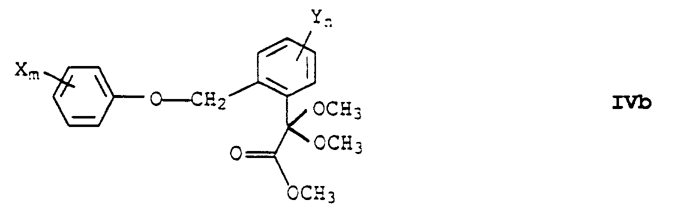 oder ein Amid der allgemeinen Formel IVc 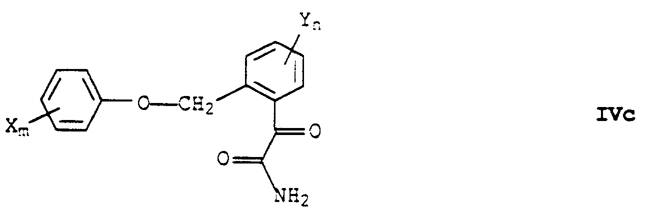 oder ein Gemisch der Verbindungen IVa, IVb und IVc mit Hydroxylamin oder einem seiner Säureadditionssalze zunächst in ein Gemisch der E- und Z-Oxime der allgemeinen Formeln IIa und IIb 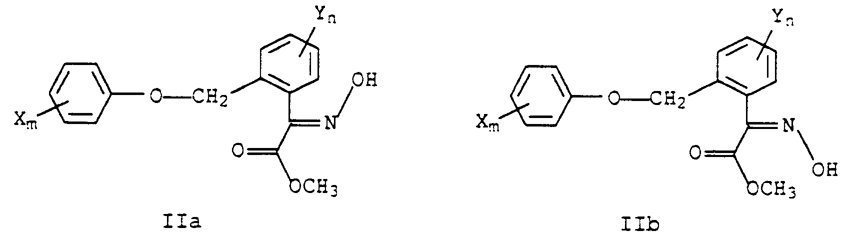 überführt, das so erhaltene Gemisch der E- und Z- Oxime anschließend durch Behandlung mit einer Säure in einem organischen Lösungsmittel in das E-Oxim der Formel IIa überführt, IIa anschließend in Gegenwart eines organischen Verdünnungsmittel mit einer Base in das entsprechende Salz überführt und dieses mit einem Methylierungsmittel der allgemeinen Formel III
CH₃X III
in der X für Chlor, Brom, Iod, CH₃OSO₃, CF₃OSO₃, (4-CH₃-C₆H₄O)-SO₃ oder (4-Br-C₆H₄O)-SO₃ steht, in I überführt.

2. Verfahren nach Anspruch 1, dadurch gekennzeichnet, daß man als Säure bei der Umlagerung des Gemisches der E- und Z- Oxime IIa und IIb Mineralsäuren, aliphatische Sulfonsäuren, aromatische Sulfonsäuren oder halogenierte Alkancarbonsäuren verwendet.

3. Verfahren nach Anspruch 1, dadurch gekennzeichnet, daß man als Säure bei der Umlagerung des Gemisches der E- und Z- Oxime IIa und IIb Chlorwasserstoff-Gas verwendet.

4. Verfahren nach Anspruch 1, dadurch gekennzeichnet, daß man die Säure bei der Umlagerung des Gemisches der E- und Z- Oxime IIa und IIb in 0,01-facher bis 10-facher molarer Menge bezogen auf die Menge des Gemisches von IIa und IIb verwendet.

5. Verfahren nach Anspruch 1, dadurch gekennzeichnet, daß man die Umlagerung des Gemisches der E- und Z- Oxime IIa und IIb bei Temperaturen von (-20°C) bis 100°C durchführt.

6. Verfahren nach Anspruch 1, dadurch gekennzeichnet, daß man für die Umlagerung des Gemisches der E- und Z- Oxime IIa und IIb einen aromatischen Kohlenwasserstoff, einem chlorierten Kohlenwasserstoff oder einem Alkohol als Lösungsmittel verwendet.

7. Verfahren nach Anspruch 1, dadurch gekennzeichnet, daß man die Umlagerung des Gemisches der E- und Z- Oxime IIa und IIb in einem 2-Phasensystem aus Wasser/Säure und einem organischen Lösungsmittel durchführt.

8. Verfahren nach Anspruch 1, dadurch gekennzeichnet, daß man die Umlagerung des Gemisches der E- und Z- Oxime IIa und IIb in einem niedermolekularen Alkohol als Lösungsmittel durchführt.

9. Verfahren nach Anspruch 1, dadurch gekennzeichnet, daß man für die Umlagerung des Gemisches der E- und Z- Oxime IIa und IIb Chlorwasserstoff-Gas als Säure und Methanol als Lösungsmittel verwendet.

## Claims

1. A process for preparing E-oxime ethers of the formula I 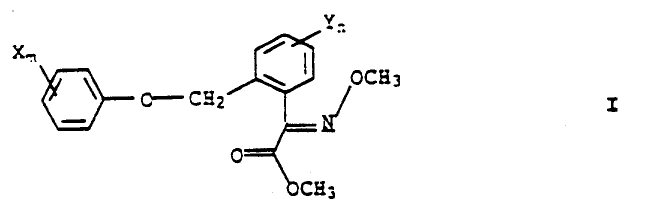 where the substituents have the following meaning:
X, Y are identical or different and are halogen, C₁-C₄-alkyl, C₁-C₄-alkoxy, trifluoromethyl, 1-C₁-C₅-alkyl-(C₂-C₅-alkenyl)hydroxyimino-C₁-C₅-alkyl (C₂-C₅-alkenyl) and 1-C₁-C₅-alkyl-(C₂-C₅-alkenyl)hydroxyimino;
m is an integer from 0 to 4;
n is an integer from 0 to 3,
which comprises first converting an o-phenoxymethylphenylglyoxylic ester of the general formula IVa 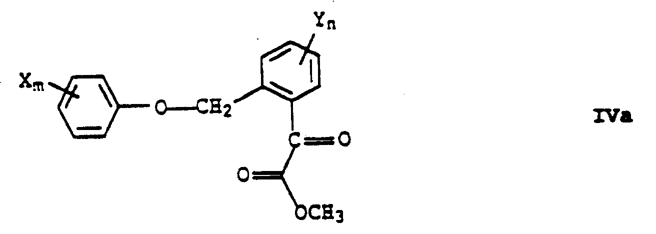 or a ketal of the general formula IVb 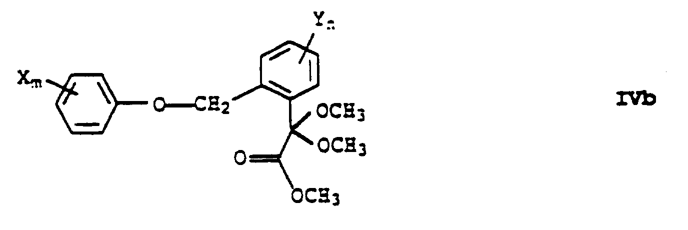 or an amide of the general formula IVc 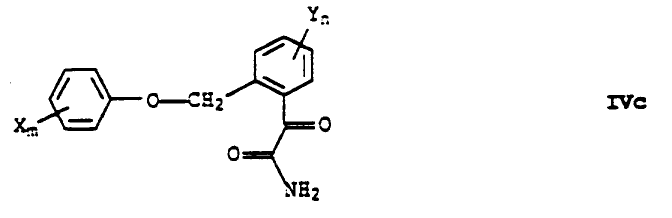 or a mixture of the compounds IVa, IVb and IVc with hydroxylamine or one or its acid addition salts into a mixture of the E- and Z-oximes of the general formulae IIa and IIb 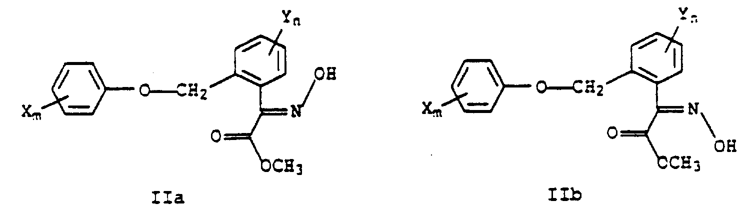 subsequently converting the resulting mixture of the E- and Z-oximes by treatment with an acid in an organic solvent into the E-oxime of the formula IIa, subsequently converting IIa in the presence of an organic diluent with a base into the corresponding salt, and converting the latter with a methylating agent of the general formula III
CH₃X III
where X is chlorine, bromine, iodine, CH₃OSO₃, CF₃OSO₃, (4-CH₃-C₆H₄O)-SO₃ or (4-Br-C₆H₄O)-SO₃ into I.

2. A process as claimed in claim 1, wherein mineral acids, aliphatic sulfonic acids, aromatic sulfonic acids or halogenated alkanecarboxylic acids, are used as acid in the rearrangement of the mixture of E- and Z-oximes IIa and IIb.

3. A process as claimed in claim 1, wherein gaseous hydrogen chloride is used as acid in the rearrangement of the mixture of E- and Z-oximes IIa and IIb.

4. A process as claimed in claim 1, wherein the acid is used in the rearrangement of the mixture of E- and Z-oximes IIa and IIb in a molar amount which is 0.01 times to 10 times the amount of the mixture of IIa and IIb.

5. A process as claimed in claim 1, wherein the rearrangement of the mixture of E- and Z-oximes IIa and IIb is carried out at from -20°C to 100°C.

6. A process as claimed in claim 1, wherein an aromatic hydrocarbon, a chlorinated hydrocarbon or an alcohol is used as solvent for the rearrangement of the mixture of E- and Z-oximes IIa and IIb.

7. A process as claimed in claim 1, wherein the rearrangement of the mixture of E- and Z-oximes IIa and IIb is carried out in a 2-phase system composed of water/acid and an organic solvent.

8. A process as claimed in claim 1, wherein the rearrangement of the mixture of E- and Z-oximes IIa and IIb is carried out in a low molecular weight alcohol as solvent.

9. A process as claimed in claim 1, wherein gaseous hydrogen chloride is used as acid and methanol is used as solvent for the rearrangement of the mixture of E- and Z-oximes IIa and IIb.

## Revendications

1. Procédé pour la préparation d'éthers d'E-oxime de formule I 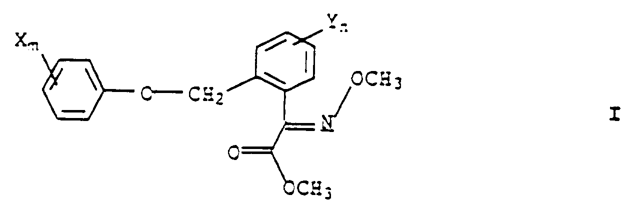 où les substituants ont la signification suivante:
X,Y sont identiques ou différents et désignent un halogène ou un groupe alkyle en C₁-C₄, alcoxy en C₁-C₄, trifluorométhyle, 1-alkyl(C₁-C₅)-alcényl(C₂-C₅)-hydroxyimino-alkyl(C₁-C₅)alcényle(C₂-C₅) et 1-alkyl(C₁-C₅)alcényl(C₂-C₅)hydroxyimino;
m représente un nombre entier de 0 à 4;
n représente un nombre entier de 0 à 3,
caractérisé par le fait qu'on convertit un ester d'acide o-phénoxyméthylphénylglyoxylique de formule générale IVa 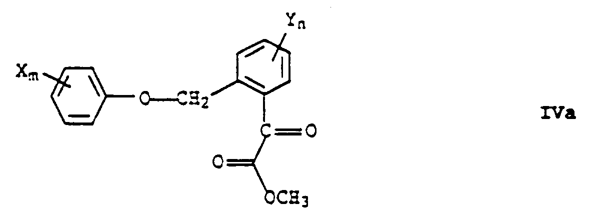 ou un cétal de formule générale IVb 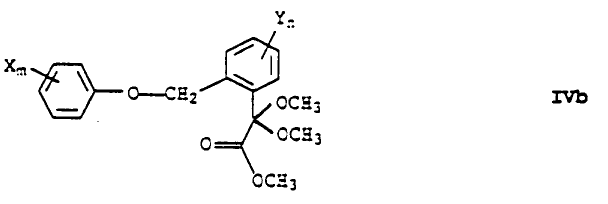 ou un amide de formule générale IVc 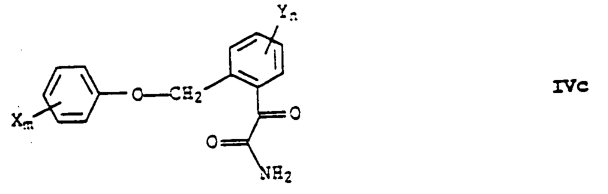 ou un mélange des composés IVa, IVb et IVc avec de l'hydroxylamine ou un de ses sels d'additon d'acide d'abord en un mélange d'oximes E et Z de formules générales IIa et IIb 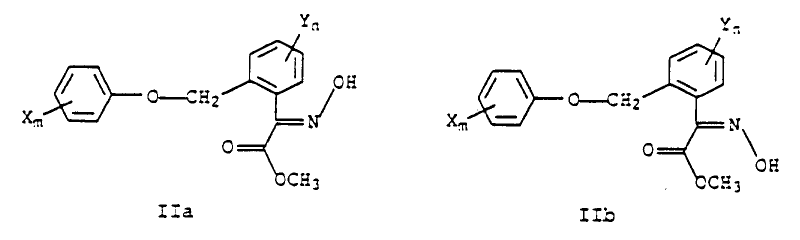 on transforme ensuite le mélange des oximes E et Z ainsi obtenu par traitement avec un acide dans un solvant organique en l'oxime E de formule IIa, on transforme ensuite IIa en présence d'un agent de dilution organique avec une base en le sel correspondant et on convertit celui-ci en I avec un agent de méthylation de formule générale III
CH₃X III
où X désigne le' chlore, le brome, l'iode, CH₃OSO₃, CF₃OSO₃, (4-CH₃-C₆H₄0)-SO₃ ou (4-Br-C₆H₄O)-SO₃.

2. Procédé selon la revendication 1, caractérisé par le fait qu'on utilise comme acide, dans la transposition du mélange des oximes E et Z IIa et IIb, des acides minéraux, des acides sulfoniques aliphatiques, des acides sulfoniques aromatiques ou des acides alcanecarboxyliques halogénés.

3. Procédé selon la revendication 1, caractérisé par le fait qu'on utilise comme acide, dans la transposition du mélange des oximes E et Z IIa et IIb, du gaz chlorhydrique.

4. Procédé selon la revendication 1, caractérisé par le fait qu'on utilise l'acide dans la transposition du mélange des oximes E et Z IIa et IIb en une quantité 0,01 fois à 10 fois molaire par rapport à la quantité du mélange de IIa et IIb.

5. Procédé selon la revendication 1, caractérisé par le fait qu'on effectue la transposition du mélange des oximes E et Z IIa et IIb à des températures de (-20°C) à 100°C.

6. Procédé selon la revendication 1, caractérisé par le fait qu'on utilise, comme solvant pour la transposition du mélange des oximes E et Z IIa et IIb, un hydrocarbure aromatique, un hydrocarbure chloré ou un alcool.

7. Procédé selon la revendication 1, caractérisé par le fait qu'on effectue la transposition du mélange des oximes E et Z IIa et IIb dans un système à 2 phases composé d'eau/acide et d'un solvant organique.

8. Procédé selon la revendication 1, caractérisé par le fait qu'on effectue la transposition du mélange des oximes E et Z IIa et IIb dans un alcool de faible masse moléculaire en tant que solvant.

9. Procédé selon la revendication 1, caractérisé par le fait qu'on utilise, pour la transposition du mélange des oximes E et Z IIa et IIb, du gaz chlorhydrique en tant qu'acide et du méthanol en tant que solvant.
